# EUROPEAN PATENT APPLICATION

(11) **EP 4 700 779 A1**
(43) Date of publication of application: **25.02.2026**
(21) Application number: 24195326.4
(22) Date of filing: 20.08.2024
(51) Int. Cl.: G16B 40/10, G16B 40/20

(54) **PRECISE AND SCALABLE MS1 QUANTIFICATION FOR DDA AND DIA USING TRANSFER LEARNING, TARGETED ANALYSIS AND SEMI-SUPERVISED MACHINE LEARNING**

(71) Applicant: Bruker Switzerland AG, 8117 Fällanden (CH)
(72) Inventor: ROSENBERGER, Georg Adrian, 8615 Wermatswil (CH); JÁUREGUI NOVO, Ignacio, 36001 Pontevedra (ES)
(74) Representative: Bremi, Tobias Hans

(57) **Abstract**

Method for the identification of analytes in a mixture using a plurality of LC-MS/MS data sets from different runs, including at least an ion mobility (IM) and a retention time (RT) dimension,
wherein in a first step, a plurality of analytes, as a subset are confidently identified individually for each run of datasets, and for each run separately, a machine learning model which learns and predicts RT and IM of said analytes, is adapted to run conditions, using transfer learning for said sampled subset of confidently identified analytes;
wherein in a second step, analytes, identified in said first step are confidently attributed in a global context over more than one run,
and wherein in a third step a global model of said machine learning model for retention time (RT) and ion mobility (IM) prediction is adapted to local conditions of each run using transfer learning, providing an query range in RT and IM dimensions for the signal processing, scoring and validation modules in a final step.

## Description

### TECHNICAL FIELD

The present invention relates to a precise and scalable MS1 quantification for DDA and DIA using transfer learning, targeted analysis and semi-supervised machine learning. Transfer learning guided peptide precursor quantification is used to replace Match-Between-Run (MBR) algorithms in medium- to large-scale datasets of bottom-up proteomic samples.

### PRIOR ART

In label-free analysis of data-dependent acquisition (DDA) datasets, different approaches have been established either based on MS1 feature finding (MS1-FF) or the extraction of ion chromatograms (MS1-XIC). Although both solutions can typically quantify the identified peptide precursors accurately within single runs, MS1-XIC-based approaches frequently have a higher recovery rate when the targeted signals are guided by peptide-precursor-defined properties such as expected retention time (RT), ion mobility (IM), and isotopic pattern. However, defining these properties based on peptide-spectrum-matches (PSMs) can be difficult, because in DDA, due to the stochastic and heuristic selection of precursors for fragmentation, the proportion of missing values dramatically increases when multiple runs are quantitatively compared. To alleviate this issue, "match-between-run" algorithms are typically employed, aligning LC gradients and transferring peptide identifications to runs with missing values. While this represents a suitable solution for small- to medium-sized sample cohorts, the approach struggles to scale to the alignment of hundreds or thousands of samples.

So, in MS-based bottom-up proteomics, data-dependent acquisition (DDA) can acquire MS/MS spectra, suitable for peptide identification, only for a fraction of all candidate peptide precursor signals. When multiple runs are quantitatively compared, the sets of identified peptides thus only partially overlap. To increase quantitative consistency across larger cohorts, the above mentioned category of algorithms has been developed that is commonly referred to as "Match-Between-Run" (MBR). The MBR algorithm assesses each identified peak in an MS1 spectrum from an LC-MS/MS run and compares its retention time to unidentified peaks in another. An identification is transferred if an unidentified peak with the same properties (e.g., m/z and charge state) is found within a specified retention time window. In a pair-wise fashion, these algorithms compare elution profiles in RT dimension to compute a relationship metric, which in turn is used to associate the closest related runs with an overlapping set of peptide identifications. For each run, the peptides, which were identified in adjacent runs, but not in the target run itself, are then aligned in retention time, ion mobility, or other dimensions for quantification without identification.

Cox J et al. in "Andromeda: A peptide search engine integrated into the MaxQuant environment", J. Proteome Res 2011, 10, 1794-1805, report that a key step in mass spectrometry (MS)-based proteomics is the identification of peptides in sequence databases by their fragmentation spectra. They describe Andromeda, a novel peptide search engine using a probabilistic scoring model. On proteome data, Andromeda performs as well as Mascot, a widely used commercial search engine, as judged by sensitivity and specificity analysis based on target decoy searches. Furthermore, it can handle data with arbitrarily high fragment mass accuracy, is able to assign and score complex patterns of post-translational modifications, such as highly phosphorylated peptides, and accommodates extremely large databases. The algorithms of Andromeda are provided. Andromeda can function independently or as an integrated search engine of the widely used MaxQuant computational proteomics platform and both are freely available at www.maxquant.org. The combination enables analysis of large data sets in a simple analysis workflow on a desktop computer. They demonstrate the flexibility of the system by implementing the capability to identify cofragmented peptides, significantly improving the total number of identified peptides.

Cox J; et al. in "Accurate proteome-wide label-free quantification by delayed normalization and maximal peptide ratio extraction, termed MaxLFQ", Mol. Cell. Proteomics 2014, 13, 2513-2526, report that protein quantification without isotopic labels has been a longstanding interest in the proteomics field. However, accurate and robust proteome-wide quantification with label-free approaches remains a challenge. They developed a new intensity determination and normalization procedure called MaxLFQ that is fully compatible with any peptide or protein separation prior to LC-MS analysis. Protein abundance profiles are assembled using the maximum possible information from MS signals, given that the presence of quantifiable peptides varies from sample to sample. For a benchmark dataset with two proteomes mixed at known ratios, they detected the mixing ratio over the entire protein expression range, with greater precision for abundant proteins. The significance of individual label-free quantifications was obtained via a t test approach. For a second benchmark dataset, they accurately quantify fold changes over several orders of magnitude, a task that is challenging with label-based methods. MaxLFQ is stated to be a generic label-free quantification technology that is readily applicable to many biological questions; it is stated to be compatible with standard statistical analysis workflows, and it stated to have been validated in many and diverse biological projects. The algorithms can handle very large experiments of 500+ samples in a manageable computing time. It is implemented in the freely available MaxQuant computational proteomics platform
and stated to work completely seamlessly.

Although these solutions are effective for small to medium-scale cohorts (10s-100s of runs), it scales poorly to large cohorts (> 1000s or runs) due to the combinatorial nature of MBR. Ludwig et al. in "Data-independent acquisition-based SWATH-MS for quantitative proteomics: a tutorial", Mol Syst Biol. 2018 Aug 13;14(8):e8126. doi: 10.15252/msb.20178126 report that many research questions in fields such as personalized medicine, drug screens or systems biology depend on obtaining consistent and quantitatively accurate proteomics data from many samples. SWATH-MS is a specific variant of data-independent acquisition (DIA) methods and is emerging as a technology that combines deep proteome coverage capabilities with quantitative consistency and accuracy. In a SWATH-MS measurement, all ionized peptides of a given sample that fall within a specified mass range are fragmented in a systematic and unbiased fashion using rather large precursor isolation windows. To analyse SWATH-MS data, a strategy based on peptide-centric scoring has been established, which typically requires prior knowledge about the chromatographic and mass spectrometric behaviour of peptides of interest in the form of spectral libraries and peptide query parameters. This tutorial provides guidelines on how to set up and plan a SWATH-MS experiment, how to perform the mass spectrometric measurement and how to analyse SWATH-MS data using peptide-centric scoring. Furthermore, concepts on how to improve SWATH-MS data acquisition, potential trade-offs of parameter settings and alternative data analysis strategies are discussed.

### SUMMARY OF THE INVENTION

Accurate relative quantification of peptides and proteins represents one of the primary applications of bottom-up mass spectrometry-based proteomics. To support a wide range of data acquisition methods with an accurate and scalable label-free quantification algorithm, we developed the approach given here. The proposed solution is particularly tailored to the analysis of timsTOF data, making use of the additional ion mobility separation dimension and state-of-the-art machine learning (ML) methods to improve quantification performance for complex samples.

So, to address the above challenges, a novel MS1-XIC-based algorithm is proposed that replaces MBR by run-wise transfer learning of global RT and IM prediction models. Using preferably the additional IM separation dimensions of timsTOF instruments provides increased specificity, while advanced isotope ion chromatogram scoring in combination with semi-supervised machine learning and statistical validation provides consistent quantification consistency with controlled error rates of quantitative features based on identified peptides and aligned missing values.

The herein described invention makes use of recent developments in deep learning-based modeling of retention time and ion mobility prediction based on peptide sequence information. Based on a single generalized model, for each run, transfer learning is conducted using a small subset of identified peptides to adapt the model to local liquid chromatography, ion mobility and sample conditions. This results in concise confidence regions where peptides are predicted to be measured and allows to transfer identifications from global to run-specific context. In combination with more sophisticated peptide precursor scoring approaches, it is shown that this approach represents a viable alternative to MBR algorithms. Most importantly, the approach scales linearly instead of exponentially with the number of runs, allowing application to cohorts spanning 1000s of samples.

### Definitions:

**LC-MS/MS:** Tandem mass spectrometry coupled to a liquid chromatography system, a technique in instrumental analysis where one or more mass analyzers are coupled together behind a liquid chromatography system using an additional reaction step to increase their abilities to analyse chemical samples.

**MS1, MS2:** The molecules of a given sample in an LC-MS/MS experiment are ionized and their mass-to-charge ratio (often given as m/z or m/Q) is measured/selected by the mass analyzer (designated MS1). Ions of a particular m/z-ratio coming from MS1 are selected and then made to split into smaller fragment ions, e.g. by collision-induced dissociation, ion-molecule reaction, or photo-dissociation. These fragments are then introduced into the mass analyzer (MS2), which in turn measures the fragments by their m/z-ratio. The fragmentation step makes it possible to identify and separate ionized molecules that have very similar m/z-ratios but produce different fragmentation patterns in MS2. The unfragmented peptide ion that dissociates to a smaller fragment ion, usually as a result of collision-induced dissociation in an MS/MS experiment, is typically referred to as precursor. Data dependent acquisition (DDA): LC-MS/MS or "shotgun" MS approach that is based on the generation of fragment ions from precursor ions that are automatically selected in the first (MS1) dimension based on the precursor ion profiles in that dimension. The window for the second (MS2) dimension is chosen as a function MS1 output (single precursor peak) automatically by the machine. This means that in this mode the MS2 dimension is not continuously sampled but only selectively as a function of the MS1 signal. In a typical shotgun acquisition method, top 10 precursor ions are selected for fragmentation per MS1 scan by the MS for measurement in MS2 with a relatively narrow isolation width of 1-2 Thomson. Precursor ions that have been selected for fragmentation are also typically ignored by the MS in the subsequent scans to allow fragmentation of new precursor ions. **Data independent acquisition (DIA):** LC-MS/MS approach, in this mode, all ionized compounds of a given sample that fall within a specified mass range in the first MS1 dimension are fragmented in a systematic and unbiased fashion resulting in corresponding spectra in the MS2 dimension. In contrast to DDA, in this case the MS2 space is continuously sampled. This not only leads to a larger data volume, but also has the effect that the spectra measured in the MS2 space comprise fragments not just from one precursor in the MS1 dimension but potentially from several such precursors. The common feature of DIA methods is that instead of selecting and sequencing a single precursor peak, wider m/z windows are fragmented resulting in complex spectra containing fragment ions of several precursors. This avoids the missing peptide ID data points typical for shotgun methods and potentially allows sequencing whole proteomes within one run, which offers a clear advantage over the small number of peptides that can be monitored per run by SRM. Furthermore, DIA have excellent sensitivity and a large dynamic range. To identify the peptides present in a sample, the fragment ion spectra can be searched against theoretical spectra or can be mined using SRM-like transitions. The detected fragments are subsequently arranged in SRM-like peak groups. In DIA acquisition, windows size in MS2 dimension is often more than 30 Thomson. This means that a typical MS2 scan in DIA is more complex than in DDA because of significantly more precursor ions being cofragmented.

**Spectrum-centric approach:** data analysis of data obtained in an LC-MS/MS experiment, which can be DDA or DIA data, in which the search is spectrum centric. This means that the spectra in the MS2 dimension are scanned for possible matches with all theoretical peptides and their fragments derived from a protein database typically with no or limited prior spectral information. Typically, the parent precursor ion for a MS2 spectrum is matched with a certain m/z tolerance to the theoretical m/z for all precursors in the search space giving a set of candidate peptides. Then the candidate peptide which best explains the spectra in terms of theoretical fragment ions is considered as the peptide spectrum match (PSM). No further prior information on the fragments is required.

**Peptide-centric approach:** data analysis of data obtained in an LC-MS/MS experiment, which can be DDA or DIA data, in which the search is precursor centric. The predicted possible peptides and their fragments derived from a predicted spectral library or an empirical spectral are queried against the spectra in the MS1 and MS2 dimension. In this analysis, spectral information of the peptides is required, in particular retention time, ion mobility, and likely to be observed fragment ions with relative fragment intensities. This information is used to narrow the search space of the peptide by querying only the spectrum that falls within a certain m/z, or IM tolerance and for scoring of matches.

**Combination-centric approach:** data analysis of data obtained in an LC-MS/MS experiment, which can be DDA or DIA data, in which the search is combination centric. Instead of identifying the best matching peptides (spectrum-centric) or testing whether particular peptides can be detected (peptide-centric), the combination centric approach employs optimization strategies to identify the set of peptides that best explain the fragment ions measured in one or several multiplexed spectra. This approach is applicable to both DDA or DIA data, where both types of spectra frequently originate from multiple cofragmented peptide species. Combination-centric strategies can be applied using theoretical or preferably using measured or predicted spectral libraries.

**Protein database:** a database, preferably selectively just for the organisms of which the sample originates, comprising peptide and protein data from these organisms, which means sequence information but no spectral information.

**Spectral library:** a database which contains information about peptide and protein systems as well as about fragments thereof, and which specifically associates to these peptides, proteins and fragments spectral information from an LC-MS/MS experiment, including (indexed) retention time, ion mobility, m/z ratios and expected fragment ion relative intensities.

**Empirical (spectral) library:** is a spectral library obtained based on an LC-MS/MS experiment typically using DDA and analysis of the data using a protein database and a spectrum-centric analysis.

**In-silico spectral library:** is a spectral library obtained using computer simulation results, such as artificial intelligence/deep learning algorithms. This type of library is also called predicted (spectral) library.

**Confident identification/attribution:** All scoring approaches defined herein generate one or several quantitative scores (e.g. cross-correlation of query and target fragment ions) that can be used to differentiate between true and false candidate signals (peptide-spectrum-matches or peak groups). Multiple scores can optionally be combined to a single score using statistical or machine learning methods, which better discriminates between the two types of signals. To assess confidence of candidates to represent a true signal, a null model based on the target-decoy-approach is used in combination with a parametric or non-parametric statistical test and multiple hypothesis correction procedures. Only candidate signals fulfilling a confidence threshold (e.g. 1% false discovery rate on peptide-spectrum-match, peptide and protein levels) are used for subsequent quantification steps. These confidence metrics can be computed for each LC-MS/MS run and in addition, for a set of related runs analyzed as part of the same experiment, which prevents accumulation of false positives in large analyses (global-context).

**Transfer learning** (TL): is a technique in machine learning (ML) in which knowledge learned from a task is re-used in order to boost performance on a related task.

According to the invention, in a first step, peptides are identified individually for each run of a dataset using any spectrum-, combination- or peptide-centric approach and DDA, DIA or DDA-pseudospectra.

In a second step, the set of identified peptides and proteins is determined in a global context, optionally grouped according to fractions or other conditions.

Third, in the MBR-replacement step, global models for RT and IM prediction are adapted to local conditions of each run using transfer learning, providing an (optionally probabilistically weighted) query range in RT and IM dimensions for the signal processing, scoring and validation modules in the final step. In contrast to linear or non-linear alignment strategies, transfer learning not only permits monotonic adjustments to be learned, but more fine-grained adaptations, including systematic changes of the elution order of individual peptides.

The proposed protocol inter alia allows to combine several approaches for a novel application:
1) use of deep learning-based predictors for RT and IM with integrated support for transfer learning.
2) use of different statistical contexts to define the query space for individual runs.
3) use of advanced XIC-based scoring algorithms and a ML-based classifier (e.g. XGBoost) to differentiate between true and false candidate signals.

Known solutions such as the approach proposed by Ivanov et al. (https://doi.orq/10.1101/2020.10.29.359075) do not use transfer learning to adapt models to local conditions, but rather uses calibration (e.g. by non-linear LOESS alignment) and do not relate to DDA applications, but rather on MS1-only accurate mass and time (AMT) datasets. Further, they do not account for the ion mobility dimension, whereas here use is made of a dedicated machine learning model for this to increase specificity.

The proposed protocol can be used as integrated component in a quantification workflow and automatically enabled once more than a single run is being analyzed. The user can see visualizations of extraction ranges in RT and IM dimensions, as well as benefit from lower running time and higher quantitative consistency.

This proposed protocol is also applicable to other omic data modalities (e.g. lipidomics, metabolomics), where the necessary predictors are available. Further, it can be extended to other separation dimensions in addition to RT and IM.

The proposed protocol can be deployed as individual component or as part of integrated data analysis solutions.

Generally speaking, the present invention relates to the method as defined in claim 1, namely to a method for the identification of analytes in a mixture, preferably for the identification of fragments of proteins and/or peptides from a sample, in particular from a digested sample, using a plurality of LC-MS/MS data sets from different runs, including at least an ion mobility (IM) and a retention time (RT) dimension.

In a first step, a plurality of analytes, preferably peptides or fragments thereof, with or without post-translational modifications, as a subset are confidently identified individually for each run of datasets, and for each run separately, a machine learning model which learns and predicts at least one of retention time (RT) and ion mobility (IM) of said analytes, preferably of peptides or fragments thereof, with or without post-translational modifications, is adapted to run conditions, using transfer learning for said sampled subset of confidently identified analytes.

In a second step, analytes, preferably peptides or fragments thereof, with or without post-translational modifications, identified in said first step are confidently attributed in a global context over more than one run, preferably a majority of runs, more preferably all runs.

In a third step a global model of said machine learning model for at least one of retention time (RT) and ion mobility (IM) prediction is adapted to local conditions of each run using transfer learning, providing a query range in at least one of retention time (RT) and ion mobility (IM) dimensions for the signal processing, scoring and validation modules in a final step.

Preferably the method is not just for identification of analytes in a mixture, but also for at least relative quantification, and in particular label-free quantification, of said analytes in the mixture.

So far, the whole field and experts have been fully focused on identification and rescoring of peptide-spectrum-matches. The idea to use the proposed approach in particular for (relative) quantification of unidentified peptide features is unexpectedly successful, because for that according to prior art approaches empirical alignment is used (match-between-run algorithms). Those may work when analysing only a handful of closely related samples, whereas the proposed approach is unexpectedly much broader and focused and allows using independent validation in particular by more complex quantitative peak scoring algorithms.

According to a preferred embodiment, using the full set of peptides confidently identified in global context in said second step, missing values not identified in run-specific context in the first step are selected and the local models are used to predict the run-specific retention time (RT) and/or ion mobility (IM) values within each run.

Using the full set of peptides confidently identified in global context in said second step, missing values not identified in run-specific context in the first step can be selected and the local models are used to estimate retention time (RT) dependent and/or ion mobility (IM) dependent window widths based on the deviation of measured and predicted values of identified peptides.

The full set of peptide precursors, their measured or predicted retention time (RT) and/or ion mobility (IM) coordinates and windows, can be used to extract precursor ion chromatograms from the MS1 scans within predefined boundaries.

According to another preferred embodiment, in the first step, a randomly sampled subset of several hundreds to thousands of confidently identified peptides is used.

In the first step, DDA, DIA or DDA-pseudospectra can be used.

Extracted ion chromatogram (XIC)-based scoring algorithms in combination with machine learning-based (e.g. eXtreme Gradient Boosting-based) classifiers, for example, support vector machines or gradient boosting, can be used to differentiate between true and false candidate signals.

According to another preferred embodiment, in said first step, said plurality of analytes, preferably peptides or fragments thereof, with or without post-translational modifications, as a subset are confidently identified individually for each run of datasets, using at least one of a spectrum-centric approach, peptide-centric approach, combination-centric approach.

In said first step, a plurality of analytes, preferably peptides or fragments thereof, with or without post-translational modifications, as a subset can be confidently identified individually for each run of datasets, and for each run separately, a machine learning model which learns and predicts retention time (RT) and ion mobility (IM) of said analytes, preferably of peptides or fragments thereof, with or without post-translational modifications, is adapted to local sample and/or instrument conditions, using transfer learning for said sampled subset of confidently identified peptides.

According to another preferred embodiment, in said second step, analytes, preferably peptides or fragments thereof, with or without post-translational modifications, identified in said first step are confidently attributed in a global context over more than one run, preferably a majority of runs, more preferably all runs, grouped according to fractions or other conditions.

According to yet another preferred embodiment, in said third step a global model of said machine learning model for retention time (RT) and ion mobility (IM) prediction is adapted to local conditions of each run using transfer learning, providing a probabilistically weighted query range in RT and IM dimensions for the signal processing, scoring and validation modules in a final step.

According to yet another preferred embodiment, as mentioned, the method is used for at least relative quantification, in particular MS1-level quantification in DDA, and in particular label-free quantification, of said analytes in the mixture, preferably by combining said machine learning model for retention time (RT) and ion mobility (IM) prediction with specific extraction spaces for quantification.

The data can be in the form of a plurality of runs of sample mass spectroscopic intensity data acquired as a function of mass to charge ratio (m/z), of retention time (RT) as well as of ion mobility (IM) determined using an LC tandem mass spectroscopy method, preferably of the TIMS type, preferably selected from the group of LC-MRM or LC-DIA.

The data can also be a set of data independent acquisition data obtained from a sample in an LC-MS/MS experiment and wherein the sample is a complex mixture of at least one protein of interest and further proteins and/or other biomolecules in the form of a complex native biological matrix which has been digested prior to LC-MS/MS analysis.

The at least one protein of interest is preferably a protein based exclusively on proteinogenic amino acids, or is based on proteinogenic amino acids and carries post-translational modifications.

Further, the invention relates to the use of a method as outlined above for the determination of at least one of the composition of the sample including quantitative and/or at least relative quantitative information about the constituents, or a medically relevant conformation of the constituents, for the determination or the influence of protein-based drugs, for the influence of drugs or other ligands on proteins, or for quality control of protein-based pharmaceutical preparations.

Further the invention relates to a computer program product to cause an LC-MS device to execute the steps of the method as given above or a computer-readable medium having stored thereon such a computer program product.

The proposed protocol represents an accurate, scalable MS1-XIC-based quantification approach, replacing MBR algorithms by state-of-the-art machine learning techniques. Together with mass accuracy and isotope assessment, ion mobility represents the most important criterion for selection of true quantitative signals.

The proposed protocol can be natively integrated within software packages, allowing the extension of Run & Done to quantitative applications.

Further embodiments of the invention are laid down in the dependent claims.

### BRIEF DESCRIPTION OF THE DRAWINGS

Preferred embodiments of the invention are described in the following with reference to the drawings, which are for the purpose of illustrating the present preferred embodiments of the invention and not for the purpose of limiting the same. In the drawings,
- Fig. 1: shows in a) an overview over the proposed workflow, in b) the details of the second step in the workflow, i.e. of the transfer learning based alignment for MBR, and in c) a workflow summary;
- Fig. 2: shows a classification of candidate signals based on XIC scores in a) and mass & ion mobility scores in b) and in c) the combined ML classifier based on the XGBoost algorithm is shown (in the bar graphs in each instance: target: left bar, decoy: right bar);
- Fig. 3: shows the quantification performance using a LFQbenchmark dataset and evaluation procedure, wherein in a) consistency of quantification events is shown (in the bar graphs: TIMS Quant: right bar) and in b) quantitative accuracy is shown.

### DESCRIPTION OF PREFERRED EMBODIMENTS

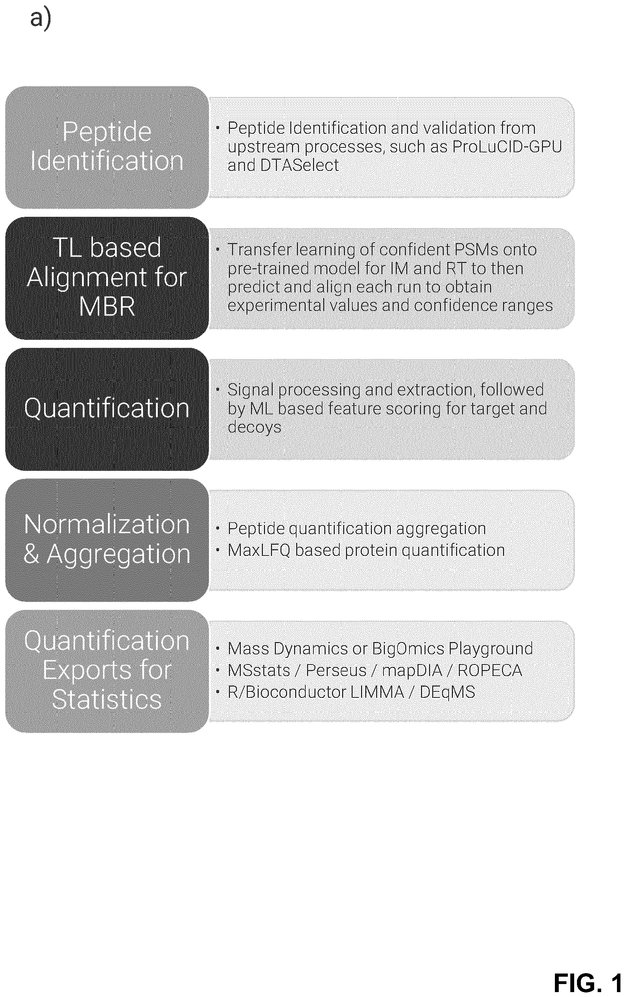

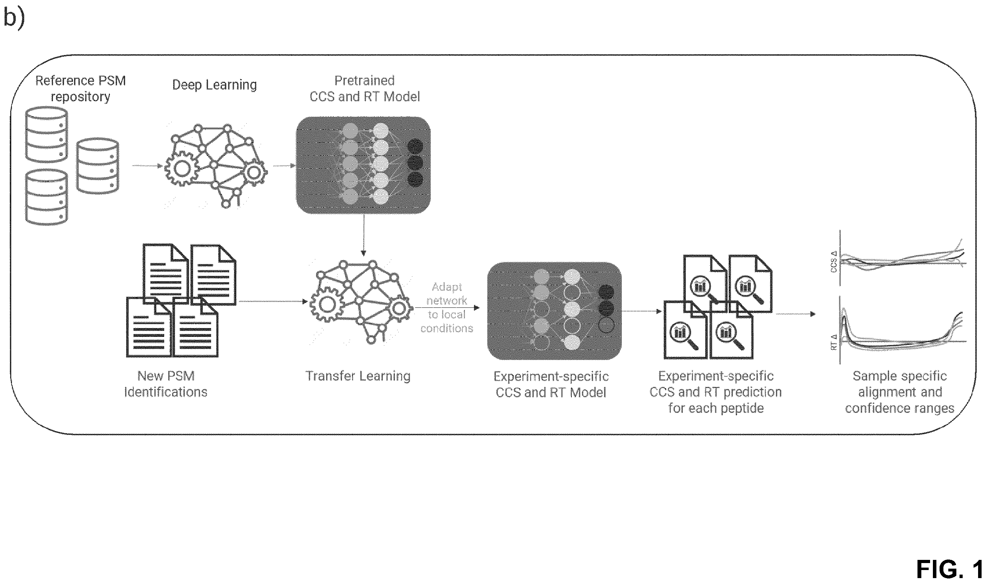

The proposed protocol uses confident Peptide-spectrum matches (PSMs) in run-wise and global contexts from upstream database search engines in addition to MS1 spectra as input (see **Fig. 1a****)**). For each run separately, global machine learning models which learn and predict the properties of peptides, with or without post-translational modifications, e.g. based on AlphaPeptDeep (Zeng et al, https://doi.org/10.1038/s41467-022-34904-3), for the prediction of RT and IM, are adapted to local sample and instrument conditions using transfer learning and a randomly sampled subset of several hundreds to thousands of confidently identified peptides (see **Fig. 1b****)**). Using the full set of peptides confidently identified in global context, the missing values not identified in run-specific context are selected and the local models are used to predict the run-specific RT and IM values within each run. In addition, RT and IM-dependent window widths based on the deviation of measured and predicted values of identified peptides are estimated.

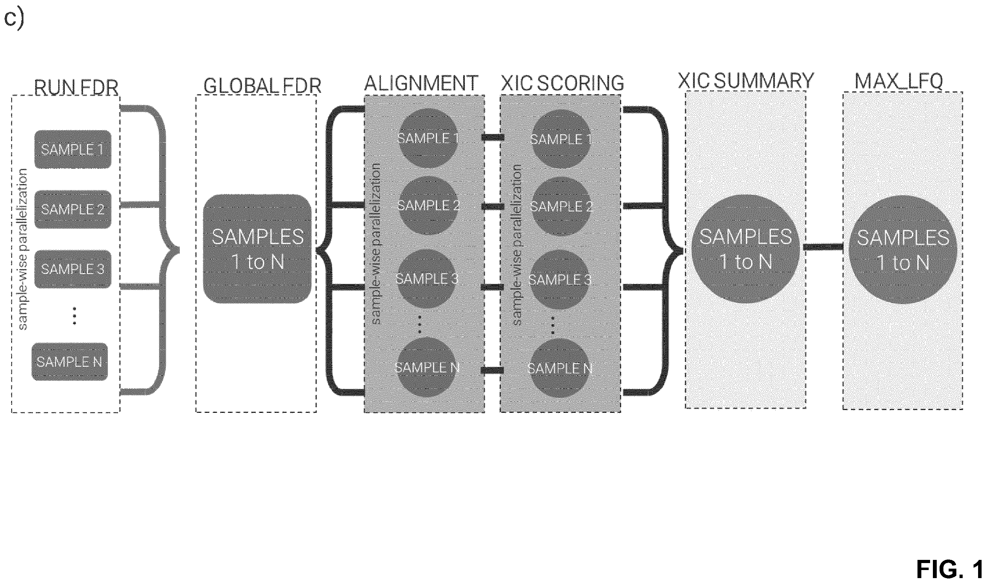

The full set of peptide precursors, their measured or predicted RT and IM coordinates and windows, are then used to extract precursor ion chromatograms from the MS1 scans within predefined boundaries (see **Fig. 1c****)**). Chromatograms are extracted for the first three isotopes within predefined mass, retention time and in mobility extraction widths. For mass and ion mobility dimensions and each theoretical precursor isotope, accuracy scores are computed that assess how close the integrated individual peaks are to the theoretical values. Chromatographic peak picking based e.g., on OpenSWATH is then used to define peak borders of the candidates using the first isotope, whereas chromatographic scoring assess cross-correlation and mutual information between isotopes.

The chromatographic scoring approach implemented in OpenSWATH (Röst et al, doi: 10.1038/nbt.2841) allows to analyze DDA (on MS1-level), SRM (on MS2-level) or DIA (on MS1- and MS2-levels) data from multiple instrument vendors. The core algorithm identifies candidate 'peak groups' (that is, positions in the chromatograms where individual fragment traces co-elute), and scores them using multiple, orthogonal scores. These scores are based on the elution profiles of the fragment ions, the correspondence of the peak group with the expected reference retention time, ion mobility and precursor isotope or fragment ion intensities.

Deviations of expected values in m/z, isotope pattern, RT and IM dimensions complement the set of scores that is derived for each candidate signal. Using an XGBoost-based semi-supervised learning approach provided by PyProphet, a classifier is trained to separate the candidate signals likely originating from true peptides from a null model derived by predicting RT and IM coordinates for mutated "decoy" peptide sequences. This classifier and the null model then allow to score and statistically validate quantitative features based on the predicted coordinates for missing values. If multiple runs are present, a supplemental peak group alignment can be performed to annotate signals that could not be confidently assigned using data from a single run alone, as described previously for DDA, SRM and DIA data.

The proposed routine exports quantitative values on peptide-precursor and protein levels using the MaxLFQ algorithm. The analysis run-time scales linearly with the number of samples and all steps can be run independently, allowing for full parallelization of the workflow.

More specifically, **Fig. 1** shows the proposed workflow, wherein in a) it is illustrated that the protocol requires identified peptides in run-specific and global contexts as input, as well as the MS1 raw data. Transfer-learning-based alignment is used as replacement for match-between-runs. Quantification is conducted by an ion chromatogram extraction (XIC)-based approach, c) illustrates how all steps of TIMSquant can be parallelized. Global context confidence estimates based on PSMs of all individual runs need to be provided to define the total set of peptides that will be quantified.

### Results

### Ion mobility increases specificity and sensitivity:

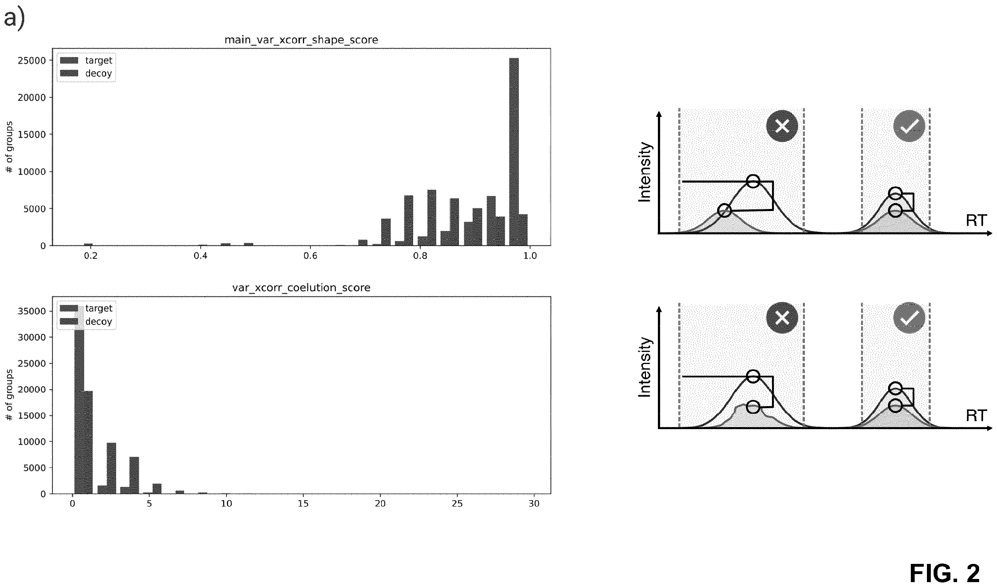

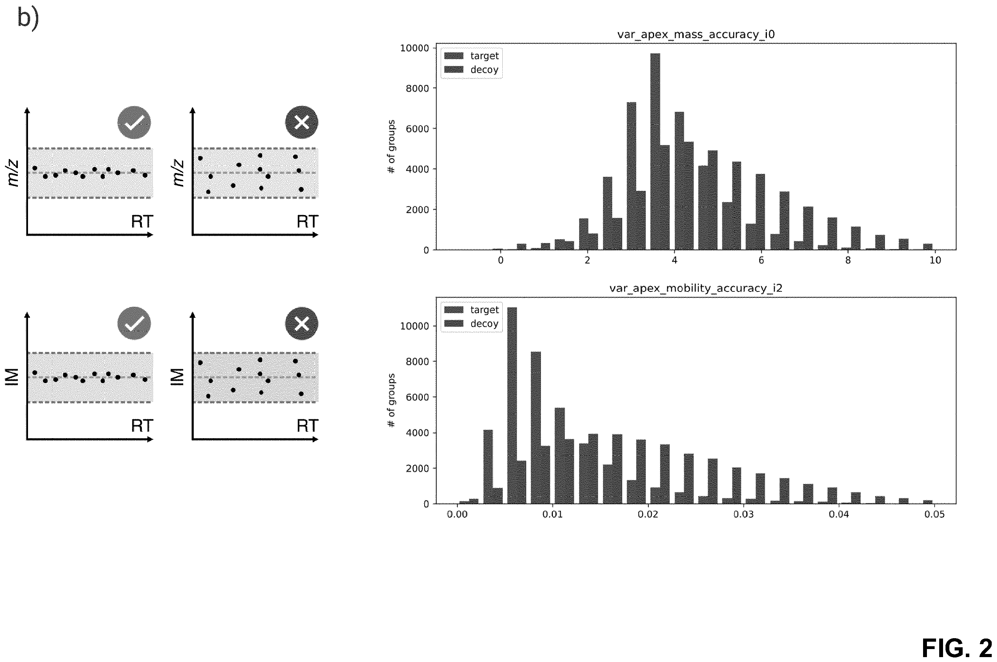

After transfer-learning-based alignment and extraction of ion chromatograms, the proposed approach computes a set of scores to assess the quality of candidate peptide signals both for identified and aligned peptides. Among the most important scores are the cross-correlation shape, cross-correlation coelution that capture the similarities of precursor isotope ion traces across the LC retention time (see **Fig. 2a****)**). In addition, mass and ion mobility accuracy typically rank among the most important scores (see **Fig. 2b****)**). On their own, these scores can be already very discriminative between true and false signals; however, combined by semi-supervised XGBoost machine learning, the combined classifier provides superior performance to each individual step.

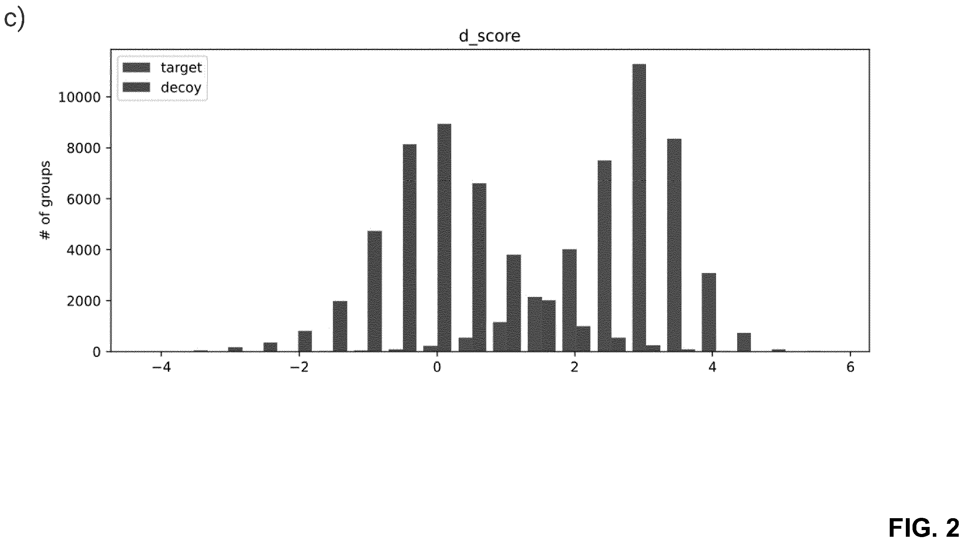

More specifically, Fig. 2 shows the classification of candidate signals based on XIC and mass & ion mobility scores. In a): cross-correlation shape and coelution scores across retention time (RT) typically are among the most discriminative scores. In b): mass and Ion Mobility (IM)-based scores provide orthogonal evidence for the correctness of candidate peptide features. In c): the combined XGBoost classifier, combining several different partial scores, provides superior performance to the individual components.

### Performance evaluation using LFQbench

To assess the performance of our method, we used the established LFQbench strategy in combination with dda-PASEF measurements of differentially mixed human, yeast, and *E.coli* samples. In total 5 replicates were measured for both Sample A and B. As reference, we used MaxQuant (2.4.20) with default parameters, with MBR enabled and disabled.

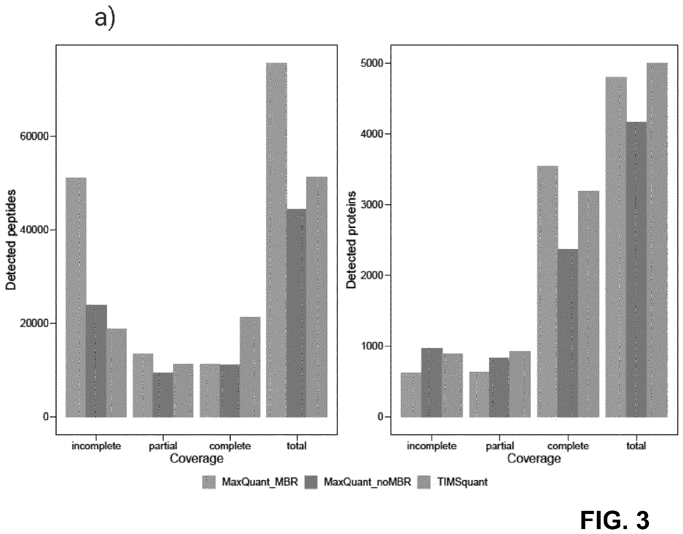

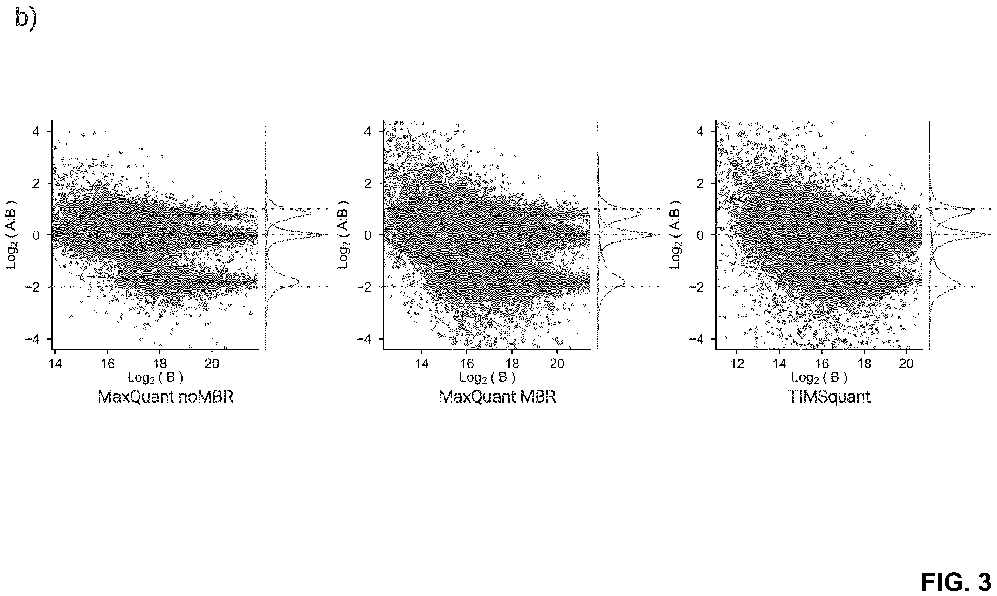

Our comparison shows that MaxQuant (without MBR) and TIMSquant quantify similar numbers of peptides (see **Fig. 3a****)).** However, the number of complete quantification events (5 quantifications in both Sample A & B) is substantially higher for the proposed approach than MaxQuant. Whereas the number of partial quantifications (5 quantifications either in Sample A or B) is similar, MaxQuant (with MBR) has many more incomplete quantification events (at least 1 quantification in Sample A or B). Assessment of quantification accuracy (see **Fig. 3b****))** suggests that the proposed approach is similarly accurate as MaxQuant (without MBR), while providing much more complete quantification across the replicates. In contrast, MaxQuant (with MBR) provides lower quantitative accuracy.

More specifically, **Fig. 3** shows the LFQbenchmark quantification performance. In a): Peptide and protein-level coverage is depicted for TIMSquant, MaxQuant (with MBR), and MaxQuant (without MBR). In b): Scatterplots and corresponding distributions suggest that the proposed approach performs similarly in terms of quantitative accuracy to MaxQuant, while providing more consistent quantification performance.

MaxQuant: MaxQuant is a comprehensive vendor-neutral bottom-up proteomic LC-MS/MS analysis suite that combines database searching using the Andromeda search engine with the original MaxQuant MS1-FF quantification algorithm. It provides an optional match-between-run methods that allow to propagate peptide identifications to runs with missing values.

dda-PASEF: On Bruker timsTOF platform instruments, data-dependent acquisition (DDA) is combined with parallel accumulation and serial fragmentation (PASEF), enabling ion mobility separated acquisition of fragment ion spectra with substantially improved coverage. ProLuCID-GPU: GPU-accelerated database search engine implementing cross-correlation-based spectrum-centric scoring using a protein database search engine as input.

DTASelect: Statistical validation approach that uses the target-decoy-approach and scores computed by ProLuCID-GPU to assign PSM, peptide- and protein-level confidence estimates.

MaxLFQ: Algorithm for quantitative protein inference using measured peptide intensities. MaxLFQ optimizes the selection and weight of peptides used to compute a measure for the aggregated protein abundance. This step is part of many proteomic data analysis suites and is typically conducted downstream of peptide-level quantification.

BigOmics Playground: A comprehensive data integration suite for the functional characterization of quantitative transcriptomic and proteomic datasets, e.g. conducting unsupervised learning (clustering), differential abundance analysis, gene set enrichment analysis and similar tasks.

MSstats: An algorithm designed for differential abundance analysis of bottom-up proteomic datasets, comparing two or more sample groups and individual replicates using a family of proteomic-optimized statistical models.

Perseus: A comprehensive data integration suite for the quantitative and functional characterization of quantitative proteomic datasets, e.g. conducting unsupervised learning (clustering), differential abundance analysis, gene set enrichment analysis and similar tasks.

mapDIA: An algorithm designed for differential abundance analysis of bottom-up proteomic datasets, comparing two or more sample groups and individual replicates using a family of proteomic-optimized statistical models.

ROPECA: An algorithm designed for differential abundance analysis of bottom-up proteomic datasets, comparing two or more sample groups and individual replicates using a family of proteomic-optimized statistical models.

PyProphet: An algorithm designed for the classification and statistical validation of chromatographic peak groups obtained for example by peptide-centric scoring. Different classifiers (e.g. LDA, SVM or XGBoost) can be trained using semi-supervised learning. Bioconductor LIMMA: An algorithm designed for differential expression analysis of transcriptomic datasets, comparing two or more sample groups and individual replicates. LFQbench: A quantitative benchmarking strategy for label-free quantification (LFQ) datasets using two differentially mixed samples containing different proteomes (e.g. human, yeast, E. coli).

DEqMS: An algorithm designed for differential abundance analysis of bottom-up proteomic datasets, comparing two or more sample groups and individual replicates using a family of proteomic-optimized statistical models.

XGBoost: eXtreme Gradient Boosting is a machine learning approach that implements a regularizing gradient boosting framework that can be used to train more robust and sensitive classifiers compared to AdaBoost, Random Forest or other methods.

### LIST OF REFERENCE SIGNS

| | | | |
|---|---|---|---|
| CCS | collision cross section | MS2 | second spectral dimension in LC-MS/MS experiment |
| DDA | Data dependent acquisition | | |
| DIA | Data-independent acquisition | RT | retention time |
| FDR | False discovery rate, the ratio between the false PSMs and the total number of PSMs above the score threshold | MS/MS | 2D mass spectroscopy with fragmentation |
| | | m/z | mass to charge ratio |
| | | PSM | Peptide-spectrum match |
| IM | Ion mobility | RT | Retention time |
| LC | Liquid chromatography | SRM | Selected Reaction Monitoring |
| LC-MS | Liquid chromatography coupled to Mass Spectrometry | TIMS | Trapped ion mobility spectrometry |
| | | timsTOF | Time-of -flight instrument coupled to a dual TIMS analyser |
| ML | Machine learning | | |
| MBR | Match between run | | |
| MRM | multiple reaction monitoring | TL | Transfer learning |
| MS | Mass spectrometry | XIC | Extracted ion chromatogram |
| MS1 | first spectral dimension in LC-MS/MS experiment | | |

## Claims

1. Method for the identification of analytes in a mixture, preferably for the identification of fragments of proteins and/or peptides from a sample, in particular from a digested sample, using a plurality of LC-MS/MS data sets from different runs, including at least an ion mobility (IM) and a retention time (RT) dimension,
wherein in a first step, a plurality of analytes, preferably peptides or fragments thereof, with or without post-translational modifications, as a subset are confidently identified individually for each run of datasets, and for each run separately, a machine learning model which learns and predicts retention time (RT) and ion mobility (IM) of said analytes, preferably of peptides or fragments thereof, with or without post-translational modifications, is adapted to run conditions, using transfer learning for said sampled subset of confidently identified analytes;
wherein in a second step, analytes, preferably peptides or fragments thereof, with or without post-translational modifications, identified in said first step are confidently attributed in a global context over more than one run, preferably a majority of runs, more preferably all runs,
and wherein in a third step a global model of said machine learning model for retention time (RT) and ion mobility (IM) prediction is adapted to local conditions of each run using transfer learning, providing a query range in retention time (RT) and ion mobility (IM) dimensions for the signal processing, scoring and validation modules in a final step.

2. Method according to claim 1, wherein, using the full set of peptides confidently identified in global context in said second step, missing values not identified in run-specific context in the first step are selected and the local models are used to predict the run-specific retention time (RT) and/or ion mobility (IM) values within each run.

3. Method according to any of the preceding claims, wherein, using the full set of peptides confidently identified in global context in said second step, missing values not identified in run-specific context in the first step are selected and the local models are used to estimate retention time (RT) dependent and/or ion mobility (IM) dependent window widths based on the deviation of measured and predicted values of identified peptides.

4. Method according to any of the preceding claims, wherein the full set of peptide precursors, their measured or predicted retention time (RT) and/or ion mobility (IM) coordinates and windows, are used to extract precursor ion chromatograms from the MS1 scans within predefined boundaries.

5. Method according to any of the preceding claims, wherein in the first step, a randomly sampled subset of several hundreds to thousands of confidently identified peptides is used.

6. Method according to any of the preceding claims, wherein in the first step, DDA, DIA or DDA-pseudospectra are used.

7. Method according to any of the preceding claims, wherein extracted ion chromatogram (XIC)-based scoring algorithms and a machine learning-based classifier to differentiate between true and false candidate signals.

8. Method according to any of the preceding claims, wherein in said first step, said plurality of analytes, preferably peptides or fragments thereof, with or without post-translational modifications, as a subset are confidently identified individually for each run of datasets, using at least one of a spectrum-centric approach, peptide-centric approach, combination-centric approach.

9. Method according to any of the preceding claims, wherein in said first step, a plurality of analytes, preferably peptides or fragments thereof, with or without post-translational modifications, as a subset are confidently identified individually for each run of datasets, and for each run separately, a machine learning model which learns and predicts retention time (RT) and ion mobility (IM) of said analytes, preferably of peptides or fragments thereof, with or without post-translational modifications, is adapted to local sample and/or instrument conditions, using transfer learning for said sampled subset of confidently identified peptides.

10. Method according to any of the preceding claims, wherein in said second step, analytes, preferably peptides or fragments thereof, with or without post-translational modifications, identified in said first step are confidently attributed in a global context over more than one run, preferably a majority of runs, more preferably all runs, grouped according to fractions or other conditions
and/or wherein in said third step a global model of said machine learning model for retention time (RT) and ion mobility (IM) prediction is adapted to local conditions of each run using transfer learning, providing a probabilistically weighted query range in RT and IM dimensions for the signal processing, scoring and validation modules in a final step
and/or wherein it is for at least relative quantification, in particular MS1-level quantification in DDA, and in particular label-free quantification, of said analytes in the mixture, preferably by combining said machine learning model for retention time (RT) and ion mobility (IM) prediction with specific extraction spaces for quantification.

11. Method according to any of the preceding claims, wherein the data is in the form of a plurality of runs of sample mass spectroscopic intensity data acquired as a function of mass to charge ratio (m/z), of retention time (RT) as well as of ion mobility (IM) determined using an LC tandem mass spectroscopy method, preferably of the TIMS type, preferably selected from the group of LC-MRM or LC-DIA.

12. Method according to any of the preceding claims, wherein the data is a set of data independent acquisition data obtained from a sample in an LC-MS/MS experiment and wherein the sample is a complex mixture of at least one protein of interest and further proteins and/or other biomolecules in the form of a complex native biological matrix which has been digested prior to LC-MS/MS analysis.

13. Method according to any of the preceding claims, wherein the at least one protein of interest is a protein based exclusively on proteinogenic amino acids, or is based on proteinogenic amino acids and carries post-translational modifications.

14. Use of a method according to any of the preceding claims for the determination of at least one of the composition of the sample including quantitative and/or at least relative quantitative information about the constituents, or a medically relevant conformation of the constituents, for the determination or the influence of protein-based drugs, for the influence of drugs or other ligands on proteins, or for quality control of protein-based pharmaceutical preparations.

15. A computer program product to cause an LC-MS device to execute the steps of the method according to any of the preceding claims 1-13 or a computer-readable medium having stored thereon such a computer program product.
